# EUROPÄISCHE PATENTSCHRIFT

(11) **EP 2 674 176 B1**
(45) Veröffentlichungstag und Bekanntmachung des Hinweises auf die Patenterteilung: **26.11.2014**
(21) Anmeldenummer: 12171667.4
(22) Anmeldetag: 12.06.2012
(51) Int. Cl.: A61M 3/02

(54) **Vorrichtung zur Einstellung des Irrigationsdruckes bei Augenoperationen**
Device for adjusting irrigation pressure during eye operations
Dispositif de réglage de la pression d'irrigation lors d'opérations oculaires

(43) Veröffentlichungstag der Anmeldung: 18.12.2013
(73) Patentinhaber: EOS GmbH, 52249 Eschweiler (DE)
(72) Erfinder: Klomp, Manfred, 4191AA Geldermalsen (NL)
(74) Vertreter: Röggla, Harald

(56) Entgegenhaltungen:
- EP-A2- 0 544 410
- EP-B1- 1 428 541
- WO-A1-2009/112251
- US-A- 4 655 197
- US-A- 5 505 707
- US-A1- 2001 023 331

## Beschreibung

Die Erfindung betrifft eine Vorrichtung zur Verwendung in der Augenchirurgie, die einen Behälter mit Fluid aufweist, der über eine Irrigationsleitung mit einem chirurgischen Handstück zum Abgeben des Fluids zum Spülen eines operierten Auges verbunden ist, wobei ein insbesondere elektrisch in seiner Höhe verstellbarer Ständer zum Tragen des Behälters und zum Verstellen der Höhe zwischen Behälter und chirurgischem Handstück vorgesehen ist, und wobei eine Steuerung zum Verstellen der Höhe des Ständers vorgesehen ist, um das Fluid mit einem vom Operateur vorgegebenen Irrigationsdruck von dem chirurgischen Handstück in das Auge abzugeben.

Das Dokument EP 1 428 541 B 1 offenbart so eine Vorrichtung, die Teil eines Augenoperationsgeräts ist, mir dem eine neue Linse in das Auge eines Patienten eingesetzt werden kann. Bei der Operation wird vorerst von dem Operateur ein Schnitt an dem Auge gesetzt, über den die in kleine Stücke zerteilte alte Linse entfernt und anschließend die neue Linse in das Auge eingebracht wird. Um das Entfernen der kleinen Stücke der alten Linse zu ermöglichen und um zu verhindern, dass das von der alten Linse ursprünglich eingenommene Volumen im Auge bei der Operation kollabiert, muss von dem chirurgischen Handstück das Fluid mit einem Irrigationsdruck in das operierte Auge abgegeben werden. Ein zu hoher Irrigationsdruck würde das Auge bleibend schädigen und bei einem zu geringen Irrigationsdruck ist die Gefahr des Kollabierens des Auges gegeben, weshalb der Operateur den geeigneten Irrigationsdruck einstellen können muss. Hierbei ist entscheidend, wie gut dem Operateur die geplante Länge des Schnitts an dem Auge gelingt, da diese das Volumen des Fluids je Zeiteinheit zum Spülen des Auges und die Druckverhältnisse während der Operation im Auge beeinflusst. Gemäß der derzeit bekannten Operationsmethoden mit der bekannten Vorrichtung wird daher nach Durchführung des Schnitts am Auge von dem Operateur einmalig die Höhe des Ständers eingestellt und somit der Irrigationsdruck der Spülflüssigkeit von dem Operateur vorgegeben.

Bei der bekannten Vorrichtung hat sich als Nachteil ergeben, dass die Höhe des Ständers nur in einem bestimmten Ausmaß einstellbar ist und bei einem besonders großen Schnitt im Auge der erforderliche Irrigationsdruck auch mit maximaler Höhe des Ständers nicht erreicht werden kann. Auch kann es sein, dass die maximal mit dem Ständer einstellbare Höhe zwar ausreichen würde, dass in dem Operationssaal aber durch bauliche Beschränkungen, wie beispielsweise der Raumhöhe, die für den gewünschten Irrigationsdruck nötige Höhe des Ständers nicht einstellbar ist. Eine weitere Vorrichtung zur Einstellung des Irrigationsdruckes ist aus US 2001/023331 A1 benannt.

Der Erfindung liegt die Aufgabe zugrunde eine Vorrichtung zur Verwendung in der Augenchirurgie zu schaffen, die dem Operateur größere Flexibilität und Sicherheit gewährleistet den für die Augenoperation nötigen und gesundheitlich unbedenklichen Irrigationsdruck des Fluids im Auge einzustellen.

Die Erfindung wird durch die Merkmale des unabhängigen Anspruchs 1 definiert. Erfindungsgemäß wird diese Aufgabestellung dadurch gelöst, dass Druckbeaufschlagungsmittel vorgesehen sind, die zum Beaufschlagen des an die Irrigationsleitung von dem Behälter abzugebenden Fluids mit einem Atmosphärenüberdruck ausgebildet sind, und dass die Steuerung zum Einstellen des gewünschten Irrigationsdrucks sowohl zum Verstellen der Höhe des Ständers als auch zum Einstellen des zu beaufschlagenden Atmosphärenüberdrucks ausgebildet ist.

Hierdurch ist der Vorteil erhalten, dass ein gewisser minimal für die Operation auf jeden Fall nötiger Irrigationsdruck des Fluids durch Beaufschlagen des Atmosphärenüberdrucks auf das Fluid mittels der Druckbeaufschlagungsmittel erreicht wird. Die Feineinstellung beziehungsweise endgültige Einstellung des gewünschten Irrigationsdrucks erfolgt durch die Steuerung durch Einstellung der Höhe des Ständers. Da ein einmal beaufschlagter Atmosphärenüberdruck nur durch das langsam abfließende Fluid oder mittels eines relativ teuren Infusionsbestecks wieder reduziert werden kann, ergibt dieser gestaffelte Aufbau des Irrigationsdrucks den Vorteil, dass durch Beaufschlagung des Atmosphärenüberdrucks ein gewisser minimaler Irrigationsdruck vorhanden ist, der mittels der einfachen und unmittelbar wirksamen Höheneinstellung des Ständers gut und zuverlässig einjustiert und auch nachgeführt werden kann.

Weitere vorteilhafte Ausgestaltungen des erfindungsgemäßen Systems werden im Folgenden anhand der Figuren näher erläutert.

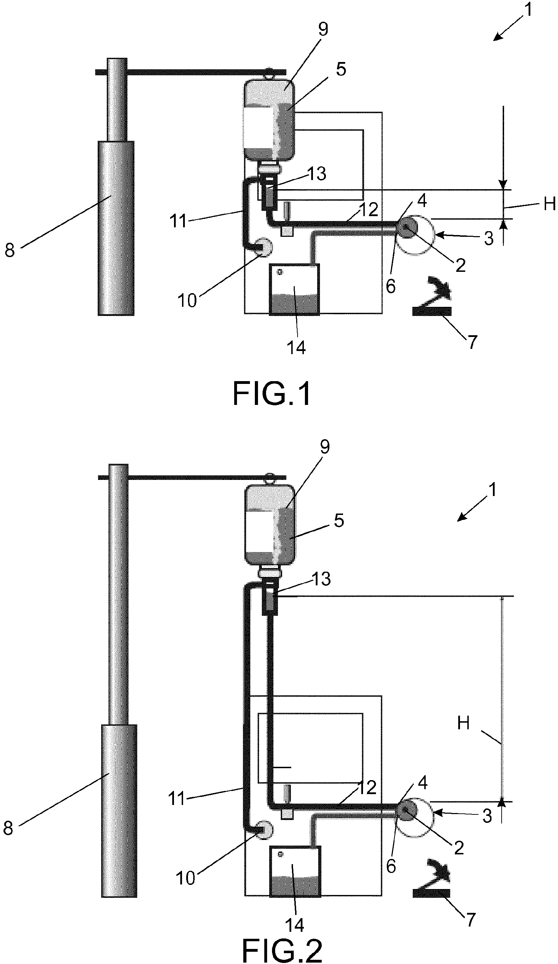

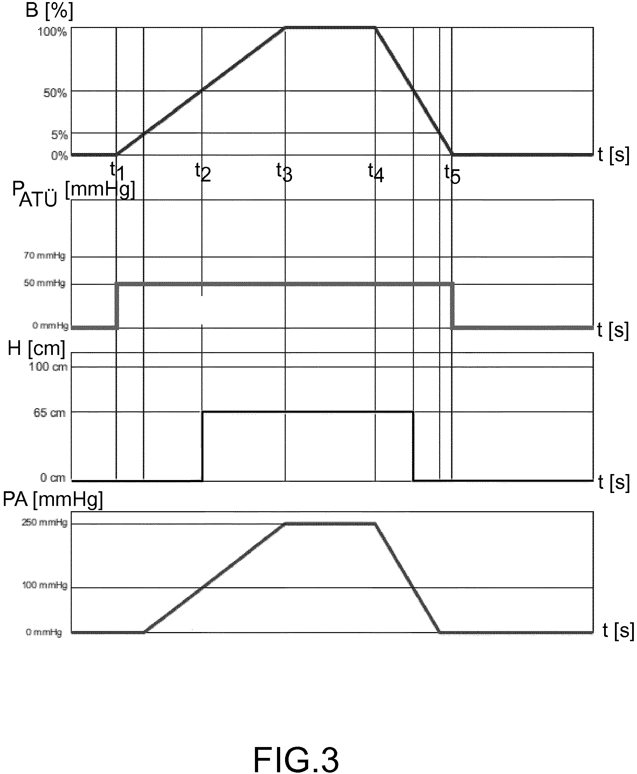

Figur 1 zeigt eine Vorrichtung zur Verwendung in der Augenchirurgie. Figur 2 zeigt die Vorrichtung gemäß Figur 1, wobei die Höhe des Ständers des Behälters verändert wurde. Figur 3 zeigt, wie die Steuerung der Vorrichtung gemäß Figur 1 den mit einem Fußschalter vorgegebenen Irrigationsdruck einstellt.

Figur 1 zeigt eine Vorrichtung 1 zur Verwendung in der Augenchirurgie, die einen Teil eines in Figur 1 nicht näher dargestellten Augenoperationsgeräts bildet. Mit dem Augenoperationsgerät kann eine neue Linse 2 in ein Auge 3 eines Patienten eingesetzt werden. Bei der Operation wird vorerst von dem Operateur ein Schnitt an dem Auge 3 gesetzt.

Ein in Figur 1 nicht näher dargestelltes chirurgisches Handstück erfüllt während der Operation drei Funktionen. An einer Irrigationsöffnung 4 wird Fluid bzw. Spülflüssigkeit 5 zum Spülen des Auges 3 von dem chirurgischen Handstück mit einem von dem Operateur vorgegebenen Irrigationsdruck in das Auge 3 abgegeben. Ein in dem chirurgischen Handstück vorgesehenes mit einem Piezo angetriebenes Messer zerteilte die alte Linse 2 in kleine Stücke, die zusammen mit der Spülflüssigkeit 5 über eine Aspirationsöffnung 6 des Handstücks eingesaugt und in einer Kassette 14 des Augenoperationsgeräts gesammelt werden.

Um das Entfernen der kleinen Stücke der alten Linse 2 zu ermöglichen und um zu verhindern, dass das von der alten Linse 2 ursprünglich eingenommene Volumen im Auge 3 bei der Operation kollabiert, muss von dem chirurgischen Handstück die Spülflüssigkeit 5 mit einem bestimmten vorgegebenem Irrigationsdruck in das operierte Auge 3 abgegeben werden. Ein zu hoher Irrigationsdruck würde das Auge 3 bleibend schädigen und bei einem zu geringen Irrigationsdruck ist die Gefahr des Kollabierens des Auges 3 gegeben, weshalb der Operateur den geeigneten Irrigationsdruck einstellen muss. Hierbei ist entscheidend, wie gut dem Operateur am Beginn der Operation die geplante Länge des Schnitts an dem Auge gelingt, da diese das Volumen der Spülflüssigkeit 5 je Zeiteinheit zum Spülen des Auges 3 beeinflusst.

Die Vorrichtung 1 weist nunmehr eine Fußschalter 7 auf, mit dem der Operateur sowohl den momentan gewünschten Irrigationsdruck als auch den momentan gewünschten Unterdruck an der Aspirationsöffnung 6 zum Absaugen vorgeben kann. Die Vorrichtung 1 weist weiters eine in den Figuren nicht näher dargestellte, durch einen Computer samt Computerprogramm der Vorrichtung 1 realisierte Steuerung auf, die, wie nachfolgend beschrieben, sowohl den vorgegebenen Irrigationsdruck an der Irrigationsöffnung 4 als auch den vorgegebenen Unterdruck an der Aspirationsöffnung 6 einstellt.

Die Vorrichtung 1 weist nunmehr zum Einstellen des Irrigationsdrucks sowohl einen in seiner Höhe elektrisch verstellbaren Ständer 8 für den Behälter 9 der Spülflüssigkeit 5, als auch Druckbeaufschlagungsmittel 10 auf, die über eine Druckleitung 11 einen Atmosphärenüberdruck auf eine Oberfläche 13 der an die Irrigationsleitung 12 abzugebenden Spülflüssigkeit 5 beaufschlagen. Die Steuerung steuert den Elektromotor des Ständers 8 an, um eine bestimmte Höhe H der Flüssigkeitssäule von dem Behälter 9 zu der Irrigationsöffnung 4 des chirurgischen Handstücks einzustellen. Die Höhe H der Flüssigkeitssäule beeinflusst den Irrigationsdruck an der Irrigationsöffnung 4. Die durch eine elektrische Druckluftpumpe gebildeten Druckbeaufschlagungsmittel 10 pumpen Druckluft mit einem von der Steuerung vorgegebenem Atmosphärenüberdruck auf die Oberfläche 13 der Flüssigkeitssäule, wodurch der Irrigationsdruck zusätzlich erhöht wird.

In Figur 3 sind vier Diagramme dargestellt, wie die Steuerung diese Vorgaben erfüllt. In dem obersten Diagramm ist eine mögliche Betätigung B des Fußschalters 7 über der Zeit t dargestellt. Bis zu einem Zeitpunkt t₁ betätigt der Operateur den Fußschalter nicht und beginnt ab diesem Zeitpunkt t₁ den Fußschalter 7 immer stärker zu betätigen, bis er zum Zeitpunkt t₂ den Fußschalter 7 zu 50% und zu einem Zeitpunkt t₃ zur Gänze, also zu 100%, durchdrückt. Von einem Zeitpunkt t₄ bis zu einem Zeitpunkt t₅ drückt der Operateur immer schwächer auf den Fußschalter 7.

In einem darunter dargestellten Diagramm ist der von der Steuerung auf Grund der Betätigung B des Fußschalters 7 durch den Operateur vorgegebene Atmosphärenüberdruck P_{ATÜ} durch die Druckbeaufschlagungsmittel 10 gemäß einem ersten Ausführungsbeispiel dargestellt. Gemäß diesem Ausführungsbeispiel wird bereits bei einem leichten Betätigen des Fußschalters 7 ab bereits 1% Betätigung B ein Druck von 50 mmHg von den Druckbeaufschlagungsmitteln 10 über die Druckleitung 11 an die Oberfläche 13 der an die Irrigationsleitung 12 abzugebenden Spülflüssigkeit 5 beaufschlagt. Dieser Atmosphärenüberdruck P_{ATÜ} wird von der Steuerung so lange aufrecht erhalten, so lange die Betätigung B des Fußschalters mehr als 1% beträgt.

In einem darunter dargestellten Diagramm ist die Verstellung der Höhe H zwischen der Oberfläche 13 in dem Behälter 9 und der Irrigationsöffnung 4 des chirurgischen Handstücks durch die Steuerung in Abhängigkeit der Betätigung B des Fußschalters 7 durch den Operateur dargestellt. Zu dem Zeitpunkt t₃ bei einer Betätigung des Fußschalters von 50% steuert die Steuerung den elektrisch verstellbaren Ständer 8 von der Höhe von H = 0cm auf die Höhe H = 65cm. Eine Höhe H = 100cm Flüssigkeitssäule entspricht etwa dem Druck von 73,5 mmHg, weshalb sich der Irrigationsdruck der Spülflüssigkeit an der Irrigationsöffnung 4 ab den Zeitpunkt t₃ aus 50 mmHg + 48 mmHg zu insgesamt 98 mmHg zusammensetzt. Dieser durch die Betätigung B des Fußschalters 7 durch den Operateur vorgegebene Irrigationsdruck wird von der Steuerung so lange aufrecht erhalten, bis der Operateur den Fußschalter 7 wieder weniger stark als 50% betätigt. Hierfür reduziert die Steuerung die Höhe H des Ständers 8.

In dem untersten Diagramm in Figur 3 ist der von einer in den Figuren nicht näher dargestellten Ansaugpumpe der Vorrichtung 1 erzeugte und durch die Steuerung gemäß diesem Diagramm gesteuerte Unterdruck PA der Aspiration dargestellt. Gemäß diesem Ausführungsbeispiel wird bei gleichmäßig zunehmender Betätigung des Fußschalters 7 von der Steuerung der Unterdruck gleichmäßig erhöht. Dies hat sich deshalb als vorteilhaft erwiesen, da bei zunehmender Betätigung des Fußschalters 7 auch das Messer in dem Handstück durch den Piezo stärker angetrieben wird und somit mehr biologisches Material anfällt, das über die Aspirationsöffnung 6 in die Kassette 14 abgesaugt werden muss.

Durch die erfindungsgemäße Steuerung zum Einstellen des gewünschten Irrigationsdrucks durch sowohl das Verstellen der Höhe H des Ständers 8 als auch durch das Beaufschlagenden der Oberfläche 13 mit dem Atmosphärenüberdruck P_{ATÜ} ist der Vorteil erhalten, dass durch Beaufschlagung des Atmosphärenüberdrucks P_{ATÜ} ein gewisser minimaler Irrigationsdruck vorhanden ist, der mittels der einfachen und unmittelbar wirksamen Höheneinstellung des Ständers 8 gut und zuverlässig einjustiert und auch nachgeführt werden kann. Je nach dem, welcher Atmosphärenüberdruck von der Steuerung vorgegeben wird, ist nur mehr eine geringe zusätzlich Druckerhöhung durch eine geringe Änderung der Höhe des Ständers 8 nötig. Hierdurch können auch bauliche Einschränkungen in dem Operationssaal den Aufbau des erforderlichen Irrigationsdrucks nicht beeinflussen.

Für den Operateur ist besonders wichtig, dass während der Operation gemäß seinen durch den Fußschalter 7 festgelegten Vorgaben die drei Funktionen des chirurgischen Handstücks zuverlässig und nachvollziehbar von der Steuerung eingestellt werden. Ein ständiges Ändern der Höhe H des Ständers 8 bei beliebig kleinen Änderungen der Betätigung B des Fußschalters 7 wäre folglich sehr störend. Es hat sich somit als vorteilhaft erwiesen den Irrigationsdruck nur durch stufenweises Erhöhen der Höhe H des Ständers 8 zu verändern, da sich der Operateur, abgesehen von den Zeitbereichen, wenn der Ständer gerade seine Höhe H ändert, auf gleichmäßige Druckverhältnisse einstellen kann, was die Operation wesentlich erleichtert.

Je nach Ausführungsbeispiel können ein oder mehrere Stufen (Vergrößerung der Höhe H des Ständers 8) bei bestimmten vorgegebenen Prozentsätzen der Betätigung B des Fußschalters 7 durch die Steuerung festgelegt sein. Ebenso könnten auch die Druckbeaufschlagungsmittel 10 vorerst nur z.B. 30 mmHg Atmosphärenüberdruck P_{ATÜ} bei z.B. B = 5% anlegen und dem Operateur so einen größeren Regelbereich mit dem Fußschalter 7 durch die Verstellung der Höhe des Ständers 8 geben. Je nach Anwendungsfall kann der Fachmann entsprechend der Erfindung die beste Zusammensetzung dieser Partialdrücke durch Atmosphärenüberdruck P_{ATÜ} und Höhenverstellung festlegen. Hierbei sind unterschiedliche Ausgestaltungen des Verfahrens zur Steuerung der Vorrichtung 1 realisierbar.

Es kann erwähnt werden, dass es bei manchen Anwendungsbeispielen auch vorteilhaft sein kann, wenn die Steuerung bei Betätigung des Fußschalters vorerst die Höhe des Ständers verstellt und erste dann mittels der Druckbeaufschlagungsmittel bei stärkerer Betätigung des Fußschalters den Irrigationsdruck weiter erhöht. Auch wäre es möglich dies abwechselnd oder in einer anderen vorgegebenen Reihenfolge durchzuführen.

Die in der Tabelle gemäß Anspruch 4 angegebenen Werte haben sich in der Praxis als besonders vorteilhaft erwiesen, um eine gute Operation der Linse des Auges zu ermöglichen.

Es kann erwähnt werden, dass in vorstehendem Ausführungsbeispiel elektrisch betriebene oder gesteuerte Funktionen auch durch pneumatisch betriebene oder gesteuerte Funktionen ersetzt werden können. So könnten beispielsweise die Druckbeaufschlagungsmittel anstatt der elektrischen Druckluftpumpe auch über eine Druckluftwand oder einen Druckluftzylinder angetrieben werden.

## Patentansprüche

1. Vorrichtung (1) zur Verwendung in der Augenchirurgie, die einen Behälter (9) mit Fluid (5) aufweist, der über eine Irrigationsleitung (12) mit einem chirurgischen Handstück zum Abgeben des Fluids (5) zum Spülen eines operierten Auges (3) verbunden ist, wobei ein elektrisch in seiner Höhe verstellbarer Ständer (8) zum Tragen des Behälters (9) und zum Verstellen der Höhe (H) zwischen Behälter (9) und chirurgischem Handstück vorgesehen ist, und wobei eine Steuerung zum Verstellen der Höhe des Ständers (8) vorgesehen ist, um das Fluid (5) mit einem vom Operateur vorgegebenen Irrigationsdruck von dem chirurgischen Handstück in das Auge (3) abzugeben, **dadurch gekennzeichnet, dass** Druckbeaufschlagungsmittel (10) vorgesehen sind, die zum Beaufschlagen des an die Irrigationsleitung (12) von dem Behälter (9) abzugebenden Fluids (5) mit einem Atmosphärenüberdruck (P_{ATÜ}) ausgebildet sind, und dass die Steuerung zum Einstellen des gewünschten Irrigationsdrucks sowohl zum Verstellen der Höhe (H) des Ständers (8) als auch zum Einstellen des zu beaufschlagenden Atmosphärenüberdrucks (P_{ATÜ}) ausgebildet ist, und dass durch einen Fußschalter (7) gebildete Vorgabemittel zum Vorgeben des Irrigationsdrucks vorgesehen sind, und dass die Steuerung bei Betätigung des Fußschalters (7) vorerst die Druckbeaufschlagungsmittel (10) zum Beaufschlagen des Atmosphärenüberdrucks (P_{ATÜ}) ansteuert und anschließen bei einer stärkeren Betätigung des Fußschalters (7) zur weiteren Erhöhung des gewünschten Irrigationsdrucks zum Verstellen der Höhe (H) des Ständers (8) ausgebildet ist.

2. Vorrichtung (1) gemäß Anspruch 1, **dadurch gekennzeichnet, dass** die Steuerung entsprechend der aktuellen Position des Fußschalters (7) zum Steuern der Druckbeaufschlagungsmittel (10) zum Beaufschlagen des Fluids (5) mit einem von zwei oder mehreren unterschiedlichen vorgegebenen Atmosphärenüberdrücken (P_{ATÜ}) ausgebildet ist und/oder, dass die Steuerung gemäß der aktuellen Position des Fußschalters (7) zum Steuern der Höhe (H) des Ständers (8) in eine von zwei oder mehreren unterschiedlichen vorgegebenen Höhen (H) ausgebildet ist.

3. Vorrichtung (1) gemäß Anspruch 2, **dadurch gekennzeichnet, dass** die Steuerung bei einer gemäß der im Folgenden angegebenen prozentualen Betätigung des Fußschalters (7) zur Steuerung der Druckbeaufschlagungsmittel (10) zur Beaufschlagung des im Folgenden angegebenen Atmosphärenüberdrucks (P_{ATÜ}) und zur Steuerung der im Folgenden angegebenen Höhe (H) zwischen Behälter (9) und chirurgischem Handstück ausgebildet ist:
| Fußschalterbetätigung (B) [%] | Atmosphärenüberdruck (P_{ATÜ}) auf Fluid [mmHg] | Höhe (H) zwischen Behälter und Handstück [cm] |
|---|---|---|
| 1 bis 10 | 30 bis 70, insbesondere 50 | 0 bis 10, insbesondere 0 |
| 10 bis 100, insbesondere 50 bis 100 | 30 bis 70, insbesondere 50 | 30 bis 100, insbesondere 60 bis 70, vorzugsweise 65 |

4. Vorrichtung (1) gemäß einem der vorstehenden Ansprüche, **dadurch gekennzeichnet, dass** eine Ansaugpumpe zum Erzeugen eines Unterdrucks (PA) vorgesehen ist, die über eine Aspirationsleitung mit dem chirurgischen Handstück zum Absaugen von Material und/oder Fluid (5) aus dem Auge (3) verbunden ist und, dass die Steuerung bei gleichmäßig zunehmender Betätigung des Fußschalters (7) zum Steuern der Ansaugpumpe zur gleichmäßigen Erhöhung des Unterdrucks (PA) und zum Steuern der Druckbeaufschlagungsmittel (10) zum stufenweisen Erhöhen des Atmosphärenüberdrucks (PATÜ) und zum stufenweisen Verstellen der Höhe (H) des Ständers (8) ausgebildet ist.

## Claims

1. A device (1) for use in eye surgery, comprising a container (9) with fluid (5) which is connected via an irrigation line (12) to a surgical handpiece for delivering the fluid (5) for rinsing an eye (3) on which surgery has been performed, wherein a pillar (8), which is electrically adjustable in its height, is provided for supporting the container (9) and for adjusting the height (H) between the container (9) and the surgical handpiece, and wherein a control for adjusting the height of the pillar (8) is provided in order to deliver the fluid (5) from the surgical handpiece into the eye (3) at an irrigation pressure predetermined by the surgeon, **characterized in that** pressurizing means (10) are provided which are designed for charging the fluid (5) to be delivered from the container (9) to the irrigation line (12) with an atmospheric overpressure (P_{ATÜ}) and that the control for adjusting the desired irrigation pressure is designed both for adjusting the height (H) of the pillar (8) and for adjusting the atmospheric overpressure (P_{ATÜ}) to be charged and that presetting means formed by a foot switch (7) are provided for presetting the irrigation pressure and that, upon actuation of the foot switch (7), the control first triggers the pressurizing means (10) in order to charge the atmospheric overpressure (P_{ATÜ}) and, upon a subsequent stronger actuation of the foot switch (7), is designed for further increasing the desired irrigation pressure in order to adjust the height (H) of the pillar (8).

2. A device (1) according to claim 1, **characterized in that** the control is designed, corresponding to the current position of the foot switch (7), for controlling the pressurizing means (10) in order to charge the fluid (5) with one of two or more different predetermined atmospheric overpressures (P_{ATÜ}) and/or that the control is designed, according to the current position of the foot switch (7), for bringing the height (H) of the pillar (8) to one of two or more different predetermined heights (H).

3. A device (1) according to claim 2, **characterized in that** the control is designed for controlling the pressurizing means (10) in order to charge the atmospheric overpressure (P_{ATÜ}) as indicated below and for controlling the height (H) between the container (9) and the surgical handpiece as indicated below, upon a percental actuation of the foot switch (7) as indicated below:
| Foot switch actuation (B) [%] | Atmospheric overpressure (P_{ATÜ}) on fluid [mmHg] | Height (H) between container and handpiece [cm] |
|---|---|---|
| 1 to 10 | 30 to 70, in particular 50 | 0 to 10, in particular 0 |
| 10 to 100, in particular 50 to 100 | 30 to 70, in particular 50 | 30 to 100, in particular 60 to 70, preferably 65 |

4. A device (1) according to any of the preceding claims, **characterized in that** a suction pump for generating a negative pressure (PA) is provided which is connected via an aspiration line to the surgical handpiece for aspirating material and/or fluid (5) from the eye (3) and that the control is designed for controlling the suction pump in order to evenly increase the negative pressure (PA) and for controlling the pressurizing means (10) in order to gradually increase the atmospheric overpressure (PATÜ) and to gradually adjust the height (H) of the pillar (8), upon a consistently increasing actuation of the foot switch (7).

## Revendications

1. Dispositif (1) à utiliser en chirurgie oculaire, qui comprend un récipient (9) avec un fluide (5), qui est relié via un conduit d'irrigation (12) à une pièce à main chirurgicale pour distribuer le fluide (5) pour rincer un oeil opéré (3), dans lequel il est prévu un support (8) déplaçable en hauteur par voie électrique destinée à supporter le récipient (9) et pour modifier la hauteur (H) entre le récipient (9) et la pièce à main chirurgicale, et dans lequel il est prévu une commande pour modifier la hauteur du support (8) afin de distribuer le fluide (5) avec une pression d'irrigation imposée par l'opérateur depuis la pièce à main chirurgicale jusque dans l'oeil (3),
**caractérisé en ce qu'**il est prévu des moyens d'application de pression (10), qui sont réalisés pour appliquer le fluide (5) à distribuer depuis le récipient (9) vers la conduite d'irrigation (12) avec une surpression atmosphérique (P_{ATM}), et **en ce que** la commande pour régler la pression d'irrigation souhaitée est réalisée aussi bien pour modifier la hauteur (H) du support (8) que pour régler la surpression atmosphérique (PATM) à appliquer, et **en ce qu'**il est prévu un moyen d'imposition, formé par un commutateur à pied (7) pour imposer la pression d'irrigation, et **en ce que** lors de l'actionnement du commutateur à pied (7) la commande pilote tout d'abord le moyen d'application de pression (10) pour appliquer la surpression atmosphérique (PATM) et ensuite sous un actionnement plus prononcé du commutateur à pied (7) pour poursuivre l'augmentation de la pression d'irrigation souhaitée, celle-ci est réalisée pour modifier la hauteur (H) du support (8).

2. Dispositif (1) selon la revendication 1, **caractérisé en ce que** la commande est réalisée pour, en correspondance de la position actuelle du commutateur à pied (7), commander le moyen d'application de pression (10) afin d'appliquer le fluide (5) avec une surpression atmosphérique (P_{ATM}) parmi deux ou plusieurs surpressions atmosphériques (P_{ATM}) prédéterminées différentes, et/ou **en ce que** la commande est réalisée pour, selon la position actuelle du commutateur à pied (7), commander la hauteur (H) du support (8) vers une hauteur (H) parmi deux ou plusieurs hauteurs prédéterminées différentes (H).

3. Dispositif (1) selon la revendication 2, **caractérisé en ce que** la commande est réalisée pour, lors d'un actionnement du commutateur à pied (7) selon les pourcentages indiqués dans ce qui suit, commander le moyen d'application de pression (10) pour appliquer la surpression atmosphérique (P_{ATM}) indiquée dans ce qui suit et pour commander la hauteur (H) indiquée dans ce qui suit entre le récipient (9) et la pièce à main chirurgicale :
| Actionnement du commutateur à pied (B) [%] | Surpression atmosphérique (PATM) sur le fluide [mmHg] | Hauteur (H) entre récipient et pièce à main [cm] |
|---|---|---|
| 1 à 10 | 30 à 70, notamment 50 | 0 à 10, notamment 0 |
| 10 à 100, notamment 50 à 100 | 30 à 70, notamment 50 | 30 à 100, notamment 60 à 70, de préférence 65 |

4. Dispositif (1) selon l'une des revendications précédentes, **caractérisé en ce qu'**il est prévu une pompe d'aspiration pour engendrer une dépression (PA), laquelle est reliée via une conduite d'aspiration à la pièce à main chirurgicale pour aspirer des matériaux et/ou du fluide (5) hors de l'oeil (3), et **en ce que** la commande est réalisée pour, lors d'un actionnement régulièrement croissant du commutateur à pied (7), commander la pompe d'aspiration pour augmenter régulièrement la dépression (PA), et pour commander le moyen d'application de pression (10) pour augmenter pas à pas la surpression atmosphérique (PATM) et pour modifier pas à pas la hauteur (H) du support (8).
